# EUROPÄISCHE PATENTSCHRIFT

(11) **EP 1 309 585 B1**
(45) Veröffentlichungstag und Bekanntmachung des Hinweises auf die Patenterteilung: **16.04.2008**
(21) Anmeldenummer: 01969522.0
(22) Anmeldetag: 26.07.2001
(51) Int. Cl.: C07D 401/12, A61K 31/47

(54) **NEUE INDOLDERIVATE UND DEREN VERWENDUNG ALS ARZNEIMITTEL**
NOVEL INDOLE DERIVATIVES AND THEIR USE AS MEDICAMENTS
NOUVEAUX DERIVES D'INDOL ET LEUR UTILISATION COMME MEDICAMENTS

(30) Priorität: 28.07.2000 DE 10037310
(43) Veröffentlichungstag der Anmeldung: 14.05.2003
(60) Teilanmeldung: 07123142.7
(73) Patentinhaber: AEterna Zentaris GmbH, 60314 Frankfurt (DE)
(72) Erfinder: EMIG, Peter, 63486 Bruchköbel (DE); BACHER, Gerald, 82110 Germering (DE); REICHERT, Dietmar, 63863 Eschau (DE); BAASNER, Silke, 61137 Schöneck (DE); AUE, Beate, 63762 Grossostheim/Ringheim (DE); NICKEL, Bernd, 64367 Mühltal (DE); GÜNTHER, Eckhard, 63477 Maintal (DE)
(86) Internationale Anmeldenummer: PCT/EP2001/008644
(87) Internationale Veröffentlichungsnummer: WO 2002/010152

(56) Entgegenhaltungen:
- EP-A- 0 490 263
- WO-A-00/26202
- WO-A-00/31063
- WO-A-00/67802
- WO-A-00/71535
- WO-A-01/12189
- WO-A-01/17516
- WO-A-01/19830
- WO-A-01/22954
- WO-A-01/47913
- WO-A-01/47916
- WO-A-01/94310
- WO-A-02/08225
- WO-A-97/22619
- WO-A-98/00946
- WO-A-99/43654
- WO-A-99/46267
- WO-A-99/51224
- WO-A-99/55696
- WO-A-99/64044
- WO-A-99/65894
- FR-M- 3 632
- US-A- 3 573 294
- EVANS S M ET AL: "PROBING THE 5-TH3 RECEPTOR SITE USING NOVEL INDOLE-3-GLYOXYLIC ACID DERIVATIVES" MEDICINAL CHEMISTRY RESEARCH, BIRKHAEUSER, BOSTON, US, Bd. 3, Nr. 5/6, 1993, Seiten 386-406, XP000956190 ISSN: 1054-2523
- DATABASE CA [Online] CHEMICAL ABSTRACTS SERVICE, COLUMBUS, OHIO, US; DA SETTIMO, A. ET AL: "Synthesis and anti-inflammatory activity of some N-(5-substituted indol-3-ylglyoxyl)amine derivatives" retrieved from STN Database accession no. 107:32773 XP002193284 & FARMACO, ED. SCI. (1987), 42(1), 17-26 ,
- BIANUCCI, ANNA MARIA ET AL: "Benzodiazepine receptor affinity and interaction of some N-(indol-3-ylglyoxylyl)amine derivatives" J. MED. CHEM. (1992), 35(12), 2214-20 , XP001056519
- DATABASE CA [Online] CHEMICAL ABSTRACTS SERVICE, COLUMBUS, OHIO, US; POLYMEROPOULOS, E. E. ET AL: "A peptidic binding site model for PDE 4 inhibitors" retrieved from STN Database accession no. 134:360953 XP002193285 & MOLECULAR MODELING AND PREDICTION OF BIOACTIVITY, [PROCEEDINGS OF THE EUROPEAN SYMPOSIUM ON QUANTITATIVE STRUCTURE-ACTIVITY RELATIONSHIPS: MOLECULAR MODELING AND PREDICTION OF BIOACTIVITY], 12TH, COPENHAGEN, DENMARK, AUG. 23-28, 1998 (2000), MEETING ,
- DATABASE CA [Online] CHEMICAL ABSTRACTS SERVICE, COLUMBUS, OHIO, US; PODWINSKI, BOHDAN: "Synthesis of some 5-benzyloxyindole-3-glyoxylic acid amides" retrieved from STN Database accession no. 70:37598 XP002193286 & ANN. ACAD. MED. LODZ. (1966), 8, 153-6 ,
- POLYMEROPOULOS E E ET AL: "A PEPTIDIC BINDING SITE MODEL FOR PDE 4 INHIBITORS" QUANTITATIVE STRUCTURE-ACTIVITY RELATIONSHIPS, VCH PUBLISHERS, DEERFIELD BEACH, FL, US, Bd. 18, Nr. 6, 1999, Seiten 543-547, XP001022210 ISSN: 0931-8771
- BATTAGLIA, SANDRA ET AL: "Indole amide derivatives: synthesis, structure-activity relationships and molecular modeling studies of a new series of histamine H1-receptor antagonists" EUR. J. MED. CHEM. (1999), 34(2), 93-105 , XP002193281
- JIANG, B. ET AL: "Syntheses and cytotoxicity evaluation of bis(indolyl)thiazole, bis(indolyl)pyrazinone and bis(indolyl)pyrazine: analogues of cytotoxic marine bis(indole) alkaloid" BIOORG. MED. CHEM. (2000), 8(2), 363-371 , XP002193282
- FAUL, MARGARET M. ET AL: "A New, Efficient Method for the Synthesis of Bisindolylmaleimides" J. ORG. CHEM. (1998), 63(17), 6053-6058 , XP002193283
- DATABASE CA [Online] CHEMICAL ABSTRACTS SERVICE, COLUMBUS, OHIO, US; SARSTEDT, BURKHARD ET AL: "Reactions with indole derivatives. XLVIII. A simple synthesis of the staurosporine aglycon" retrieved from STN Database accession no. 98:215863 XP002193287 & HETEROCYCLES (1983), 20(3), 469-76 ,
- DATABASE CA [Online] CHEMICAL ABSTRACTS SERVICE, COLUMBUS, OHIO, US; POVALYAEVA, O. S. ET AL: "Synthesis and properties of N-substituted 4-amino-1,2-dithiolanes and related compounds" retrieved from STN Database accession no. 101:72646 XP002193288 & ZH. ORG. KHIM. (1984), 20(4), 849-60 ,
- DATABASE CA [Online] CHEMICAL ABSTRACTS SERVICE, COLUMBUS, OHIO, US; AFSAH, EL SAYED M. ET AL: "Synthesis and reactions of N-indol-3-ylmethylalkylamines and related compounds" retrieved from STN Database accession no. 102:6101 XP002193289 & J. CHEM. SOC., PERKIN TRANS. 1 (1984), (8), 1929-32 ,
- DATABASE CA [Online] CHEMICAL ABSTRACTS SERVICE, COLUMBUS, OHIO, US; JIANG, BIAO ET AL: "Total Synthesis of (.+-.)-Dragmacidin: A Cytotoxic Bis(indole)alkaloid of Marine Origin" retrieved from STN Database accession no. 121:280941 XP002193290 & J. ORG. CHEM. (1994), 59(22), 6823-7 ,
- DATABASE CA [Online] CHEMICAL ABSTRACTS SERVICE, COLUMBUS, OHIO, US; MARTINI, CLAUDIA ET AL: "Specific inhibition of benzodiazepine receptor binding by some N-(indol-3-ylglyoxylyl)amino acid derivatives" retrieved from STN Database accession no. 102:125088 XP002193291 & J. MED. CHEM. (1985), 28(4), 506-9 ,
- BACHER GERALD ET AL: "D-24851, a novel synthetic microtubule inhibitor, exerts curative antitumoral activity in vivo, shows efficacy toward multidrug-resistant tumor cells, and lacks neurotoxicity" CANCER RESEARCH, AMERICAN ASSOCIATION FOR CANCER RESEARCH, BALTIMORE, MD, US, Bd. 61, Nr. 1, 2001, Seiten 392-399, XP002173885 ISSN: 0008-5472
- DATABASE CA [Online] CHEMICAL ABSTRACTS SERVICE, COLUMBUS, OHIO, US; FRESNEDA, PILAR M. ET AL: "Microwave-assisted regioselective synthesis of 2,4-disubstituted imidazoles: nortopsentin D synthesized by minimal effort" retrieved from STN Database accession no. 134:281011 XP002193292 & SYNLETT (2001), (2), 218-221 ,

## Beschreibung

Die Erfindung betrifft neue Indol-Derivate der allgemeinen Formel 1, deren Herstellung und Verwendung als Arzneimittel, insbesondere zur Behandlung von Tumoren.

Indol-3-yl-Derivate mit bestimmten 2-, 3-, 4-, und 8-Chinolin-Resten sind in der deutschen Anmeldung DE 198 14 838.0 beschrieben. Indol-3-yl-Derivate mit 5-, 6- oder 7-Chinolin-Substituenten sind dort aber weder beschrieben noch nahegelegt.

Gemäß einem Aspekt der Erfindung werden neue Indol-Derivate der allgemeinen Formel 1 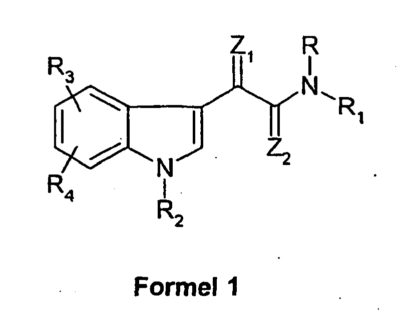 worin
- R: Wasserstoff; geradkettig oder verzweigtes (C₁ -C₆)-Alkyl; ein- oder mehrfach durch Aryl substituiertes (C₁ -C₆)-Alkyl, wobei der Arylrest unsubstituiert oder ein- bis fünffach gleich oder verschieden mit Halogen, (C₁ -C₆)-Alkyl, (C₃ -C₇)-Cycloalkyl, Carboxyl; (C₁ -C₆)-Alkoxycarbonyl, vorzugsweise tertiärButoxycarbonyl, mit einem oder mehreren Fluoratomen substituiertes geradkettig oder verzweigtes (C₁ -C₆)-Alkyl, vorzugsweise Trifluormethyl, Hydroxy, geradkettig oder verzweigtes (C₁ -C₆)-Alkoxy, vorzugsweise Methoxy oder Ethoxy; Benzyloxy, oder Aryl-(C₁ -C₆)-Alkyl, wobei der Arylrest unsubstituiert oder ein- oder fünffach gleich oder verschieden mit (C₁ -C₆)-Alkyl, Halogen oder mit einem oder mehreren Fluoratomen substituiertes geradkettig oder verzweigtes (C₁ -C₆)-Alkyl, vorzugsweise Trifluormethyl, substituiert sein kann; geradkettig oder verzweigtes (C₁ -C₆)-Alkoxy-(C₁ -C₆)-Alkyl, substituiertes oder unsubstituiertes Aryl-(C₁ -C₆)-alkyloxycarbonyl, vorzugsweise Benzyloxycarbonyl, geradkettig oder verzweigtes (C₁ -C₆)-Alkyloxycarbonyl, geradkettig oder verzweigtes (C₁ -C₆)-Alkylcarbonyl, vorzugsweise Acetyl, (C₂ -C₆)-Alkenyl, vorzugsweise Allyl, (C₂ -C₆)-Alkinyl, vorzugsweise Ethinyl oder Propargyl, oder geradkettiges oder verzweigtes Cyano-(C₁ -C₆)-Alkyl, vorzugsweise Cyanomethyl, bedeutet;
- R₁: einen 2-, 3-, 4-, 5-, 6-, 7- oder 8-Chinolyl-Rest oder 2-, 3-, 4-, 5-, 6-, 7- oder 8-Chinolyl-(C₁ -C₄)-alkyl-Rest, wobei der (C₁ -C₄)-alkyl-Rest unsubstituiert oder ein- oder mehrfach gleich oder verschieden mit (C₁ -C₆)-Alkyl, Halogen oder Oxo (=O) substituiert sein kann und der 2-, 3-, 4-, 5-, 6-, 7- oder 8-Chinolyl-Rest unsubstituiert oder ein- bis sechsfach gleich oder verschieden mit Wasserstoff, (C₁-C₆)-Alkyl, vorzugsweise Methyl, besonders bevorzugt 2-Methyl, Halogen, Nitro, Amino, Mono-(C₁-C₆)-Alkylamino, N,N-di-(C₁-C₆)-Alkylamino, Hydroxyl, (C₁-C₆)-Alkoxy, (C6-C14)-Aryl-(C1-C6)-alkyoxy, vorzugsweise Benzyloxy, Carboxy, (C₁-C₆)-Alkoxycarbonyl, (C₁-C₆)-Alkoxycarbonylamirio oder ein- oder mehrfach mit Halogen substituiertem (C₁-C₆)-Alkyl, vorzugsweise Trifluormethyl, (C₆-C₁₄)-Aryl, oder (C₆-C₁₄)-Aryl-(C₁-C₆)-alkyl substituiert sein kann, *ausgenommen mit* (C1-C6)-Alkyl, Halogen, Nitro, Amino, und (C1-C6)-Alkylamino substituiertes oder unsubstituiertes 2-, 3-, 4- und 8-Chinolyl und 2-, 3- und 4-Chinolylmethyl, bei welchem die Ringkohlenstoffatome des Pyridylmethyl-Teils unsubstituiert oder mit (C1-C6)-Alkyl, (C1-C6)-Alkoxy, Nitro, Amino, und (C1-C6)-Alkoxycarbonylamino substituiert sind, bedeutet;
- R₂: Wasserstoff, (C₁-C₆)-Alkyl, ein- oder mehrfach mit Halogen substituiertes (C₁-C₆)-Alkyl, ein- oder mehrfach mit Phenyl substituiertes (C₁-C₆)-Alkyl, wobei der Phenylrest unsubstituiert oder ein- bis fünffach gleich oder verschieden mit Halogen, vorzugsweise Chlor, Brom oder Jod, (C₁-C₆)-Alkyl, (C₃-C₇)-Cycloalkyl, Carboxyl, (C₁-C₆)-Alkoxycarbonyl, ein- oder mehrfach mit Halogen substituiertem (C₁-C₆)-Alkyl, vorzugsweise Trifluormethyl, Hydroxy, (C₁-C₆)-Alkoxy, vorzugsweise Methoxy oder Ethoxy, Phenyl-(C₁-C₆)-Alkoxy, vorzugsweise Benzyloxy, Nitro, Amino, Mono-(C₁-C₆)-alkylamino, Di-(C₁-C₆)-alkylamino, Mono-(C₃-₆)-cycloalkylamino, Di-(C₃-₆)-cycloalkylamino, (C₁-C₆)-Acylamino, Phenyl-(C₁-C₆)-alkylamino, Arylcarbonylamino, Heteroarylcarbonylamino, (C₁-C₆)-Alkylsulfonamido, Arylsulfonamido Maleinimido, Succinimido, Phthalimido, Benzyloxycarbonylamino (Z-Amino), tert.-Butoxycarbonylamino (BOC-Amino), 9-Fluorenylmethoxy-carbonylamino (Fmoc-Amino), Triphenylmethylamino (Tr-Amino), 2-(4'-Pyridyl)-ethoxycarbonylamino (Pyoc-Amino), Diphenylmethylsilyl-amino (DPMS-Amino), mit -NH-CH₂-COOH; -NH-CH(CH₃)-COOH; (CH₃)₂CH-CH₂-CH₂-CH(NH-)-COOH; H₃C-CH₂-CH(CH₃)-CH(NH-)-COOH;
HOH₂C-CH(NH-)-COOH; Phenyl-CH₂-CH(NH-)-COOH; (4-Imidazolyl)-CH₂-CH(NH-)-COOH; HN=C(NH₂)-NH-(CH₂)₃-CH(NH-)-COOH; H₂N-(CH₂)₄-CH(NH-)-COOH;
H₂N-CO-CH₂-CH(NH-)-COOH; HOOC-(CH₂)₂-CH(NH-)-COOH substituiert sein kann;
einen 2-, 3-, 4-, 5-, 6-, 7- und 8-Chinolyl-(C₁-C₆)-Alkyl-Rest, wobei der 2-, 3-, 4-, 5,-, 6- , 7- und 8-Chinolyl-Rest unsubstituiert oder ein- bis sechsfach gleich oder verschieden mit Halogen, (C₁-C₄)-Alkyl oder (C₁-C₄)-Alkoxy substituiert sein kann;
einen 2-, 3- und 4-Pyridyl-(C₁-C₆)-Alkyl-Rest, wobei der 2-, 3- und 4-Pyridyl-Rest unsubstituiert oder ein- bis vierfach gleich oder verschieden mit Halogen, (C₁-C₄)-Alkyl oder (C₁-C₄)-Alkoe substituiert sein kann;
einen Arylcarbonyl-Rest, wobei der Aryl-Rest unsubstituiert oder ein- bis fünffach gleich oder verschieden mit Halogen, (C₁-C₆)-Alkyl, (C₃-C₇)-Cycloalkyl, Carboxyl, Cyano, (C₁-C₆)-Alkoxycarbonyl, ein- oder mehrfach mit Fluoratomen substituiertes (C₁-C₆)-Alkyl vorzugsweise Trifluormethyl, Hydroxyl, (C₁-C₄)-Alkoxy vorzugsweise Methoxy oder Ethoxy, Aryl-(C₁-C₄)-Alkoxy, vorzugsweise Benzyloxy, substituiert sein kann; bedeutet;
- ₃ und R₄: R können an die Indolkohlenstoffatome C-2, C-4, C-5, C-6 oder C-7 gebunden sein und unabhängig voneinander Wasserstoff, Hydroxy, (C₁-C₆)-Alkyl, (C₃-C₇)-Cycloalkyl, (C₁-C₆)-Alkylcarbony), (C₁-C₆)-Alkoxy, Halogen, Aryl-(C₁-C₄)-Alkoxy, vorzugsweise Benzyloxy, Nitro, Amino, Mono-(C₁-C₄)-Alkyl-amino, Di-(C₁-C₄)-Alkylamino, (C₁-C₆)-Alkoxycarbonylamino, (C₁-C₆)-Alkoxycarbonylamino-(C₁-C₆)-alkyl, Cyano, geradkettiges oder verzweigtes Cyano-(C₁-C₆)-alkyl, Carboxyl, (C₁-C₄)-Alkoxycarbonyl, mit ein oder mehreren Fluoratomen substituiertes (C₁-C₄)-Alkyl, vorzugsweise Trifluormethylgruppe, Carboxy-(C₁-C₆)-alkyl oder (C₁-C₆)-Alkoxycarbonyl-(C₁-C₆)-alkyl bedeuten;
- Z₁: Sauerstoff oder Schwefel oder geminal verknüpftes Wasserstoff und Hydroxy bedeutet;
- Z₂: Sauerstoff oder Schwefel bedeutet,
deren Tautomere, Stereoisomere, einschließlich der Diastereomere und Enantiomere, sowie die physiologisch verträglichen Salze davon, bereitgestellt.

So lassen sich beispielsweise die erfindungsgemäßen Verbindungen gemäß der allgemeinen Formel (1), welche ein oder mehrere Chiralitätszentren aufweisen und die als Racemate auftreten, nach an sich bekannten Methoden in ihre optischen Isomeren, also Enantiomere oder Diastereomere auftrennen. Die Trennung kann durch Säulentrennung an chiralen Phasen oder durch Umkristallisation aus einem optisch aktiven Lösungsmittel oder unter Verwendung einer optisch aktiven Säure oder Base oder durch Derivatisierung mit einem optisch aktiven Reagenz, wie beispielsweise einem optisch aktiven Alkohol, und anschließender Abspaltung des Restes erfolgen.

Des weiteren können die erfindungsgemäßen Indol-Derivate der allgemeinen Formel (1) in ihre Salze mit anorganischen oder organischen Säuren, insbesondere für die pharmazeutische Anwendung in ihre physiologisch verträglichen Salze, überführt werden. Als Säuren kommen hierfür beispielsweise Salzsäure, Bromwasserstoffsäure, Schwefelsäure, Phosphorsäure, Fumarsäure, Bernsteinsäure, Milchsäure, Äpfelsäure, Embonsäure, Malonsäure, Zitronensäure; Essigsäure, Weinsäure, Trifluoressigsäure, Methansulfonsäure, Sulfoessigsäure oder Maleinsäure in Betracht.

Außerdem lassen sich die erfindüngsgemäßen Verbindungen gemäß der Formel (1), falls diese eine ausreichend saure Gruppe wie eine Carboxygruppe enthalten, gewünschtenfalls in ihre Salze mit anorganischen oder organischen Basen, insbesondere für die pharmazeutische Anwendung in ihre physiologisch verträglichen Salze, überführt werden. Als Basen kommen hierbei beispielsweise Natriumhydroxid, Kaliumhydroxid, Calciumhydroxid, Lysin, Cyclohexylamin, Ethanolamin, Diethanolamin und Triethanolamin in Betracht.

Gemäß einer weiteren Ausführungsform werden Indolderivate gemäß der allgemeinen Formel (1) bereitgestellt, dadurch gekennzeichnet, dass R, R2, R3, R4, Z1 und Z2 die vorstehend beschriebenen Bedeutungen besitzen und R₁ für Chinolyl steht, welches ein oder mehrfach mit geradkettig oder verzweigtem (C₁-C₆-Alkyl) oder (C₁-C₆)-Alkoxy substituiert ist.

Gemäß einer weiteren Ausführungsform werden Indolderivate gemäß der allgemeinen Formel (1) bereitgestellt, dadurch gekennzeichnet, dass R, R2, R3, R4, 21 und Z2 die vorstehend beschriebenen Bedeutungen besitzen und R, für 2-Methyl-6-chinolyl steht.

Gemäß einer weiteren Ausführungsform werden Indolderivate gemäß der allgemeinen Formel (1) bereitgestellt, dadurch gekennzeichnet, dass R, R2, R3, R4 Z1 und Z2 die vorstehend beschriebenen Bedeutungen besitzen und R₁ (2-Methyl)-6-chinolyl, R₂ 4-Chlorbenzyl, R₃ und R₄ jeweils Wasserstoff und Z₁ und Z₂ jeweils Sauerstoff bedeuten.

Gemäß einem weiteren Aspekt der Erfindung wird ein Verfahren zur Herstellung von Indolderivaten gemäß der allgemeinen Formel (1) nach einem der Ansprüche 1 bis 4, dadurch gekennzeichnet, dass eine Indolvorstufe der allgemeinen Formel (2), 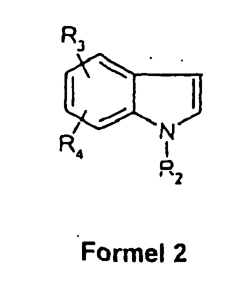 worin R2, R3 und R4 die vorstehend genannten Bedeutungen besitzen, mit einer Verbindung der allgemeinen Formel (3) 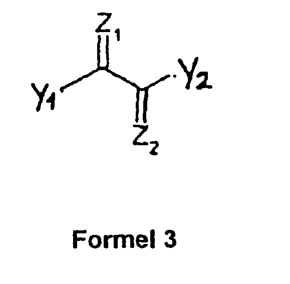 worin Z1 und Z2 die vorstehend genannte Bedeutung besitzen und Y1 und Y2 unabhängig voneinander für eine geeignete Abgangsgruppe wie Halogen, (C1-C6)-Alkoxy, -O-Tosyl, -O-Mesyl oder- N1-imidazol steht, und anschließend mit einem Amin der allgemeinen Formel (4) oder (5), 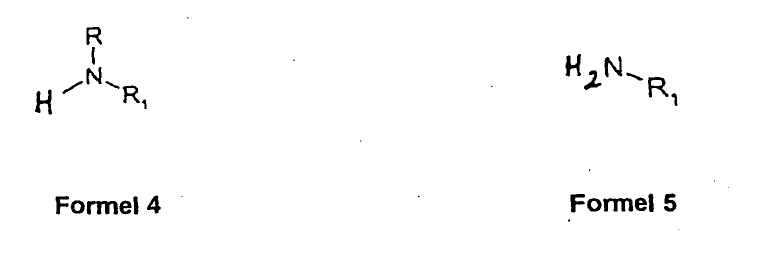 worin R und R1 die vorstehend genannten Bedeutungen haben, unter Bildung des gewünschten Indolderivates der allgemeinen Formel (1) (mit Amin 4) oder der Verbindung der allgemeinen Formel (6) (mit Amin 5) 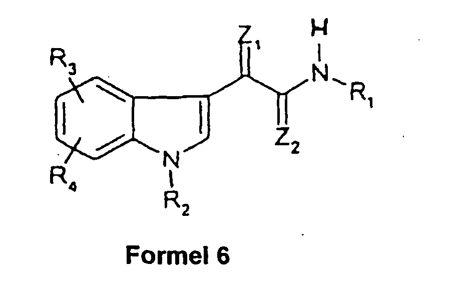 worin R1, R2, R3, R4, Z1 und Z2 die vorstehend genannten Bedeutungen besitzen, gegebenenfalls unter Verwendung von Verdünnungs- und Hilfsmitteln umgesetzt wird, wobei anschließend die Verbindung der allgemeinen Formel (6) mit einer Verbindung der allgemeinen Formel (7)

R-Y3 (7)

worin R die vorstehend genannte Bedeutung besitzt und Y3 für eine geeignete elektrophile Abgangsgruppe wie Halogen, (C1-C6)-Alkoxy, -O-Tosyl, -O-Mesyl oder-N1-imidazol steht, unter Erhalt der gewünschten Verbindung (1), wobei R nicht Wasserstoff bedeutet, gegebenenfalls unter Verwendung von Verdünnungs- und Hilfsmitteln umgesetzt werden kann.

### Synthesewege:

Die Verbindungen der allgemeinen Formel 1 sind gemäß des folgenden Schemas 1 erhältlich:

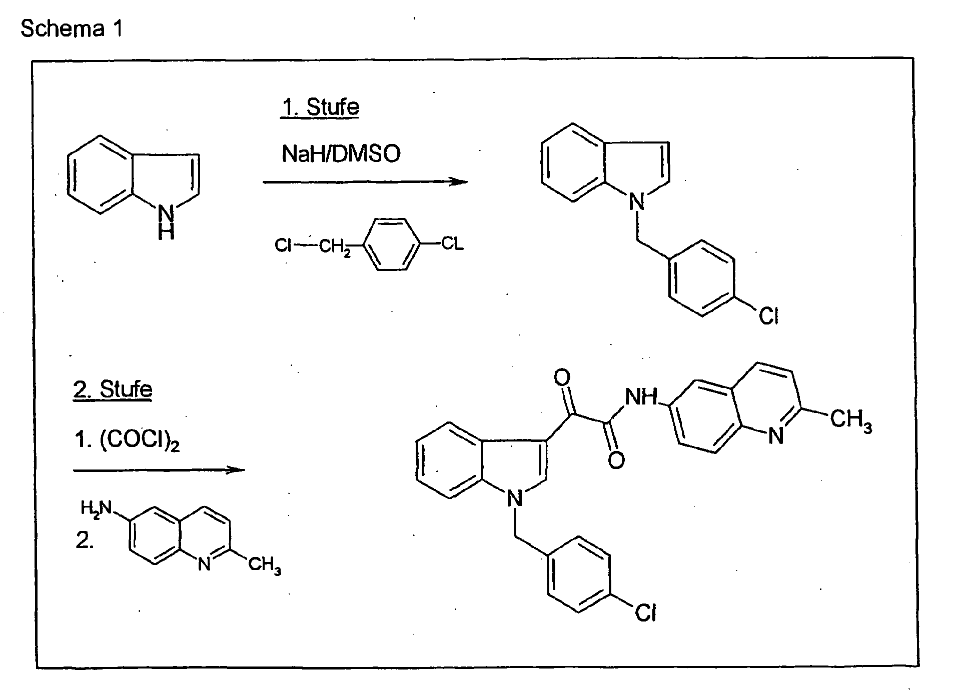

Weiterhin sind die Verbindungen der allgemeinen Formel 1 auch noch nach dem Syntheseweg des Schemas 2 erhältlich:

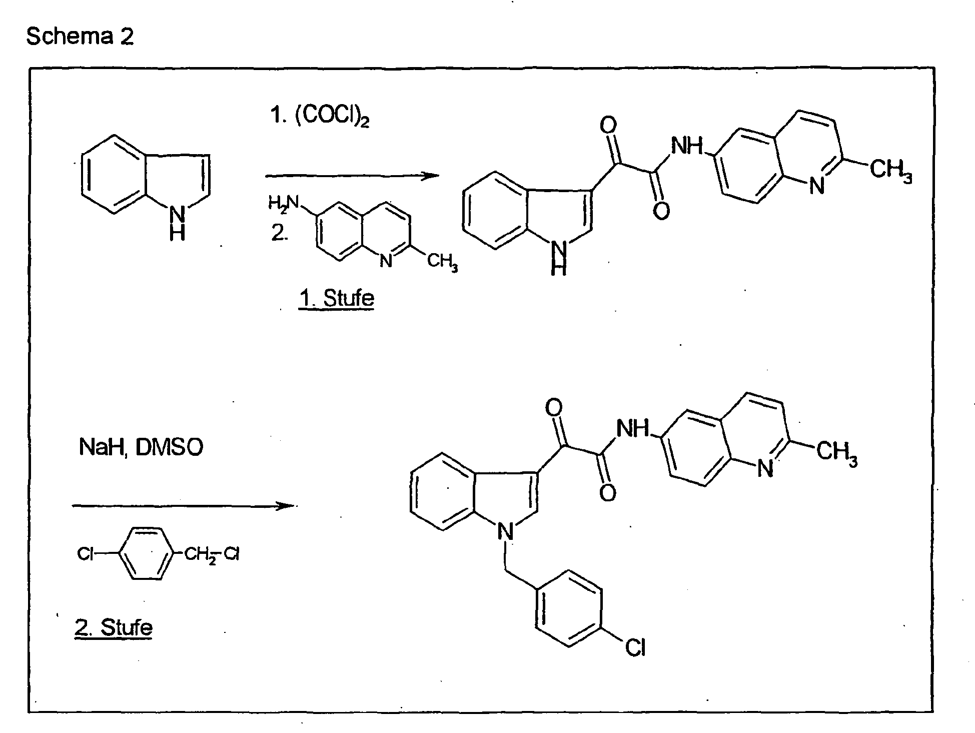

Die Verbindungen der allgemeinen Formel 1 bei denen R= die Methylgruppe, der Benzylrest, die Propargylgruppe oder der Cyanomethyl-Rest ist und R₁, R₂, R₃ und R₄ der allgemeinen Formel 1 die angegebenen Bedeutungen besitzen, können auch nach dem Syntheseweg des Schemas 3 hergestellt werden:

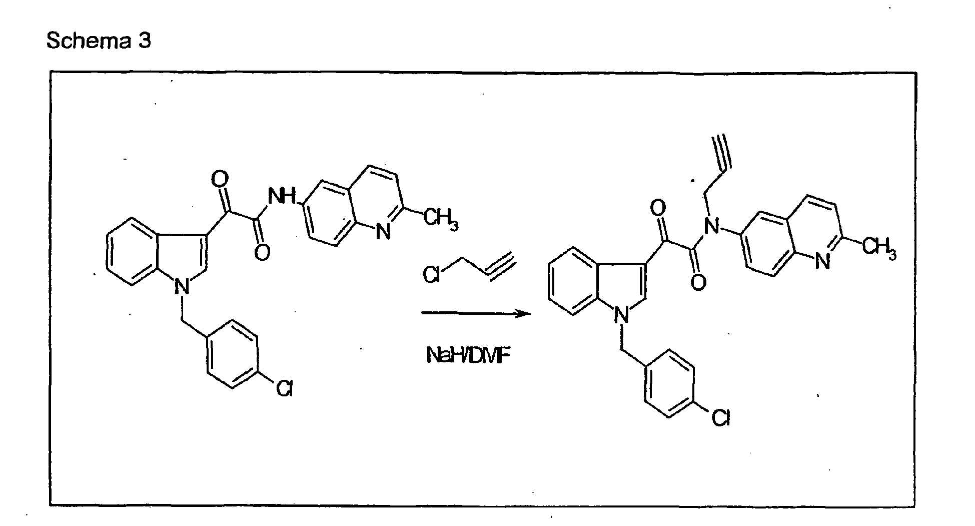

Die Ausgangsverbindungen (2), (3) und (4) sind entweder im Handel erhältlich oder können nach an sich bekannten Verfahrensweisen hergestellt werden. Die Edukte (2), (3) und (4) stellen wertvolle Zwischenverbindungen für die Herstellung der erfindungsgemäßen Indolderivate der Formel (1) dar.

Für die Herstellung der Ausgangs- und Zielverbindungen sei beispielsweise auf folgende Standardwerke der organischen Synthese verwiesen, dessen Inhalt hiermit Bestandteil der Offenbarung der vorliegenden Anmeldung werden soll:
1) Houben-Weyl, Band E 7a (Teil1) S. 290-492, S.571-740
   Houben-Weyl, Band E 7a (Teil 2) S. 119-156, S.205-686, S. 157-204
2) Monographie "Heterocyclic Compounds" (Elderfield), Volume 1, S.119- 207, S. 397-616
   Volume 3, S. 1-274
   Volume 6, S. 101-135, S. 234-323
3) Monographie "Comprehensive Organic Chemistry" (S.D.Barton, W.D. Ollis) Volume 4, S.155-204, S.205-232, S.493-564

Die gegebenenfalls zu verwendenden Lösungs- und Hilfsmittel und anzuwendenden Reaktionsparameter wie Reaktionstemperatur und -dauer sind dem Fachmann aufgrund seines Fachwissens bekannt.
Die erfindungsgemäßen Indolderivate gemäß der allgemeinen Formel (1) sind als Arzneimittel, insbesondere als Antitumormittel, zur Behandlung von Säugetieren, insbesondere dem Menschen, aber auch für Haustiere wie Pferde, Kühe, Hunde, Katzen, Hasen, Schafe, Geflügel und dergleichen geeignet.

Die Indol-Derivate werden in einem Verfahren zur Bekämpfung von Tumoren in Säugetieren, insbesondere beim Menschen bereit gestellt, welches dadurch gekennzeichnet ist, dass mindestens ein Indol-Derivat gemäß der allgemeinen Formel (1) einem Säugetier in einer für die Tumorbehandlung wirksamen Menge verabreicht wird. Die für die Behandlung zu verabreichende therapeutisch effektive Dosis des jeweiligen erfindungsgemäßen Indol-Derivates richtet sich u.a. nach der Art und dem Stadium der Tumorerkrankung, dem Alter und Geschlecht des Patienten, der Art der Verabreichung und der Dauer der Behandlung. Die Verabreichung kann oral, rectal, buccal (z.B. sublingual), parenteral (z.B. subkutan, intramuskulär, intradermal oder intravenös), topisch oder transdermal erfolgen.

Gemäß einem weiteren Aspekt der Erfindung werden Arzneimittel zur Tumorbehandlung bereitgestellt, welche dadurch gekennzeichnet sind, dass sie als wirksamen Bestandteil mindestens ein Indol-Derivat nach einem der Ansprüche 1 bis 4 oder einem pharmazeutisch verträglichen Salz davon, gegebenenfalls zusammen mit üblichen pharmazeutisch verträglichen Hilfs-, Zusatz- und Trägerstoffen enthalten. Es kann sich dabei um festen, halbfeste, flüssige oder Aerosol-Zubereitungen handeln. Geeignete feste Zubereitungen sind beispielsweise Kapseln, Pulver, Granulate, Tabletten. Geeignete halbfeste Zubereitungen sind beispielsweise Salben, Cremes, Gele, Pasten, Suspensionen, Öl-in-Wasser- und Wasser-in-Öl-Emulsionen. Geeignete flüssige Zubereitungen sind beispielsweise sterile wäßrige Zubereitungen für die parenterale Verabreichung, die isoton mit dem Blut des Patienten sind.

Die Erfindung soll anhand des nachfolgenden Beispiels näher erläutert werden, ohne darauf beschränkt zu sein.

### Ausführungsbeispiele

### Beispiel 1 (Umsetzung gemäß Schema 1, 1. Stufe):

### Herstellung von 1-(4-Chlorbenzyl)-indol

In eine Mischung von 1,32 g Natriumhydrid (0,055 Mol, Mineralölsuspension) in 50 ml Dimethylsulfoxid wird eine Lösung von 5,86 g (0,05 Mol) Indol in 25 ml DMSO gegeben. Man erhitzt 1,3 Stunden auf 60°C, läßt danach abkühlen und tropft 17,7 g (0,11 Mol) 4-Chlor-benzylchlorid zu. Man erwärmt die Lösung auf 60°C, läßt über Nacht stehen und gießt sodann unter Rühren in 200 ml Wasser. Man extrahiert mehrmals mit insgesamt 75 ml CH₂Cl₂, trocknet die organische Phase mit wasserfreiem Natriumsulfat, filtriert und engt das Filtrat im Vakuum ein.
Ausbeute: 11,5g (95% d. Th.)

### Beispiel 2 (Umsetzung gemäß 2. Stufe von Schema 1):

### Herstellung von N-(2-Methyl-6-chinolyl)-[1-(4-chlorbenzyl)-indol-3-yl]-glyoxylsäureamid (D-69429)

Zu einer Lösung von 5,50 ml Oxalylchlorid in 50 ml Ether wird bei 0°C und unter Stickstoff tropfenweise eine Lösung von 10,2 g (42,2 mmol) 1-(4-Chlorbenzyl)-indol in 50 ml Ether gegeben. Man erhitzt 2 Stdn. zum Rückfluss und dampft anschließend das Lösungsmittel ab. Sodann wurden zum Rückstand 100 ml Tetrahydrofuran zugefügt, die Lösung auf -4°C gekühlt und tropfenweise mit einer Lösung von 15,66 g (99,0 mmol) 6-Amino-2-methyl-chinolin in 350 ml THF versetzt. Man erhitzt 4 Stunden am Rückfluß und läßt über Nacht bei Raumtemperatur stehen. Das 6-Amino-2-methyl-chinolin Hydrochlorid wird abgesaugt, der Niederschlag mit THF gewaschen, das Filtrat im Vakuum eingeengt und der Rückstand aus Methylethylketon/Methylenchlorid umkristallisiert.
Ausbeute: 14,8 g (77,3% d.Th.)
Schmelzpunkt: 182-185°C

### Herstellung des Hydrochlorids:

Zu 0,453 g (1 mMol) N-(2-Methyl-6-chinolyl)-[1-(4-chlorbenzyl)-indol-3-yl]-glyoxylsäureamid in 20 ml heißem Ethanol wird die equivalente Menge isopropanolische Salzsäure gegeben, das Gemisch auf 70-80°C erhitzt und die Lösung i. Vak. bei 40°C zur Trockne eingeengt. Mehrmaliges Nachdampfen mit Toluol ergab das farblose und kristalline Hydrochlorid.
Ausbeute: 0,49 g (100% d. Th.)
Fp.:196°C

### Herstellung des Methansulfonats:

Ein Gemisch von 0,453 g (1 mMol) N-(2-Methyl-6-chinolyl)-[1-(4-chlorbenzyl)-indol-3-yl]-glyoxylsäureamid und 0,67 ml Methansulfonsäure in 15 ml Dichlormethan wird 30 Minuten auf 50°C erwärmt, die gebildete Lösung i. Vak. bei 35°C eingeengt, der Rückstand mehrmals mit Methyl-tert.-butylether nachgedampft und der verbleibende Rückstand bei 35°C i.Vak. getrocknet.
Ausbeute: 0,46 g (84 % d.Th.)
Fp.: > 230°C

### Beispiel 3 (Umsetzung gemäß Schema 3):

### Herstellung von N-Propargyl-N-(2-methyl-6-chinolyl)-[1-(4-chlorbenzyl)-indol-3-yl]-glyoxylsäureamid

Zu einer Suspension von 0,154 g Natriumhydrid (5,13 mMol, Mineralölsuspension) in 10 ml DMF wird bei Raumtemperatur und unter Stickstoff eine Suspension von 2,32 g (5,13 mmol) N-(2-Methyl-6-chinolyl))-[1-(4-chlorbenzyl)-indol-3-yl]-glyoxylsäureamid in 20 ml DMF gegeben. Es erfolgte unter Gelbfärbung ein starkes Aufschäumen. Man tropft eine Lösung von 0,382 g (5,13 mMol) Propargylchlorid in 10 ml DMF zu, rührt 24 Stdn. bei Raumtemperatur unter Stickstoff-Begasung und läßt 4 Tage bei Raumtemperatur stehen. Die dunkelbraune Lösung wird sodann auf 120 ml Eiswasser gegossen, die Lösung portionsweise mit 250 ml Methylenchlorid extrahiert und die vereinigten organischen Phasen mit wasserfreiem Natriumsulfat getrocknet. Man engt i. Vak. ein, reinigt den Rückstand über eine Kieselgelsäule (Kieselgel 60, Fa. Merck AG, Darmstadt) unter Anwendung des Elutionsmittels Methylenchlorid/Ethanol (97:3, V/V).
Ausbeute: 1,98 g (78,6% d. Th.)
Mass spectrum: m/e= 491,9 (M⁺)

### Biologische Untersuchungen

### 1. Anti-proliferative Wirkung an verschiedenen Tumor Zelllinien

Die Substanz D-69429 wurde in einem Proliferationstest an etablierten Tumorzelllinien auf ihre anti-proliferative Aktivität hin untersucht. Der verwendete Test bestimmt die zelluläre Dehydrogenase Aktivität und ermöglicht eine. Bestimmung der Zellvitalität und indirekt der Zellzahl. Bei den verwendeten Zelllinien handelt es sich um die humane Cervixkarzinom Zellinie KB / HeLa (ATCC CCL17), die murine lymphozytäre Leukämie L1210 (ATCC CCL-219), die humane Brustadenokarzinomlinie MCF7 (ATCC HTB22) und die ovariale Adenokarzinomlinie SKOV-3 (ATCC HTB77). Es handelt sich hierbei um sehr gut charakterisierte, etablierte Zelllinien, die von ATCC erhalten und in Kultur genommen wurden.

Die in Tab. 1 gezeigten Ergebnisse belegen eine sehr potente anti-proliferative Wirkung von D-69429 an den Zelllinien SKOV-3, L-1210 und HeLa/KB. Aufgrund der Besonderheit des langsamen Wachstums der MCF7 Linie ist die Wirkung von D-69429 im Versuchszeitraum von 48h nur gering (18% Hemmung bei 3.16 µg/ml; daher Angabe >3.16).

### 2. Methode

### XTT-Test auf zelluläre Dehydrogenase-Aktivität

Die adherent wachsenden Tumorzelllinien HeLa/KB, SKOV-3 und MCF7 sowie die in Suspension wachsende L1210 Leukämielinie wurden unter Standardbedingungen im Begasungsbrutschrank bei 37°C, 5% CO₂ und 95% Luftfeuchtigkeit kultiviert. Am Versüchstag 1 werden die adherenten Zellen mit Trypsin / EDTA abgelöst und durch Zentrifugation pelletiert. Nachfolgend wird das Zellpellet im RPMI Kulturmedium in der entsprechenden Zellzahl resuspendiert und in eine 96-well Mikrotiterplatte umgesetzt. Die Platten werden dann über Nacht im Begasungsbrutschrank kultiviert. Die Testsubstanzen werden als Stammlösungen in DMSO angesetzt und am Versuchstag 2 mit Kulturmedium in den entsprechenden Konzentrationen verdünnt. Die Substanzen in Kulturmedium werden dann zu den Zellen gegeben und für 45h im Begasungsbrutschrank inkubiert. Als Kontrolle dienen Zellen, die nicht mit Testsubstanz behandelt werden.

Für das XTT-Assay werden 1 mg/ml XTT (Natrium 3'-[1-(phenylaminocarbonyl)-3,4-tetrazolium]-bis(4-methoxy-6-nitro)benzensulfonsäure) in RPMI-1640 Medium ohne Phenolrot gelöst. Zusätzlich wird eine 0,383 mg/ml PMS (N-Methyl Dibenzopyrazine Methylsulfat) Lösung in Phosphat-gepufferter Salzlösung (PBS) hergestellt. Am Versuchstag 4 wird auf die Zellplatten, die inzwischen 45 h mit den Testsubstanzen inkubiert wurden, 75µl/well XTT-PMS-Mischung pipettiert. Dazu wird kurz vor Gebrauch die XTT-Lösung mit der PMS-Lösung im Verhältnis 50:1 (Vol:Vol) gemischt. Anschließend werden die Zellplatten im Begasungsbrutschrank für weitere 3h inkubiert und im Photometer die optische Dichte (OD₄₉₀ₙₘ) bestimmt.

Mittels der bestimmten OD₄₉₀ₙₘ wird die prozentuale Hemmung relativ zur Kontrolle berechnet. Die anti-proliferative Wirkung wird mittels einer Regressionsanalyse abgeschätzt.

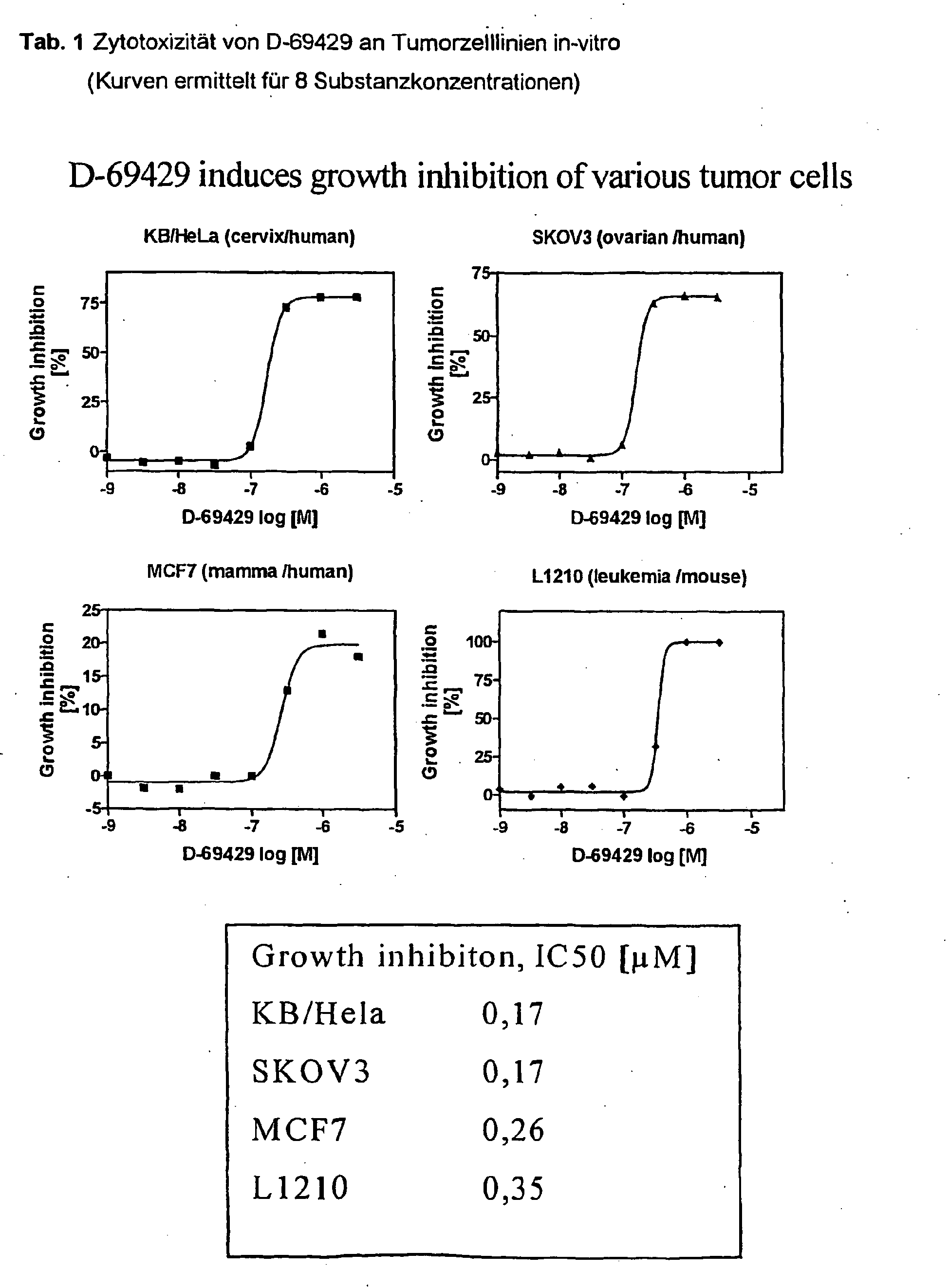

### Beispiel I

Tablette mit 50 mg Wirkstoff

### Zusammensetzung:

| | |
|---|---|
| (1) Wirkstoff | 50, 0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| Summe: | 215,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt.

### Beispiel II

Kapsel mit 50 mg Wirkstoff

### Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| Summe: | 160,0 mg |

### Herstellung:

(1) wird mit (3) verrieben. Diese Verreibung wird der Mischung aus (2) und (4) unter intensiver Mischung zugegeben. Diese Pulvermischung wird auf einer Kapselabfüllmaschine in Hartgelatine-Steckkapseln Größe 3 abgefüllt.

## Patentansprüche

1. Indol-Derivate der allgemeinen Formel 1 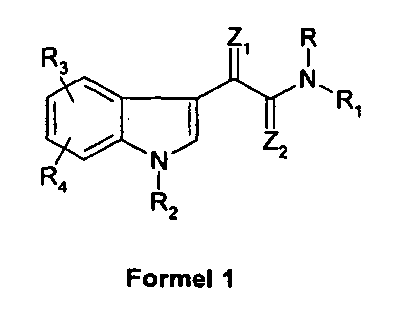 worin
R Wasserstoff; geradkettig oder verzweigtes (C₁ -C₆)-Alkyl; ein- oder mehrfach durch Aryl substituiertes (C₁ -C₆)-Alkyl, wobei der Arylrest unsubstituiert oder ein- bis fünffach gleich oder verschieden mit Halogen, (C₁ -C₆)-Alkyl, (C₃ -C₇)-Cycloalkyl, Carboxyl, (C₁ -C₆)-Alkoxycarbonyl, mit einem oder mehreren Fluoratomen substituiertes geradkettig oder verzweigtes (C₁ -C₆)-Alkyl, Hydroxy, geradkettig oder verzweigtes (C₁-C₆)-Alkoxy, Benzyloxy, oder Aryl-(C₁ -C₆)-Alkyl, wobei der Arylrest unsubstituiert oder ein- oder fünffach gleich oder verschieden mit (C₁ -C₆)- Alkyl, Halogen oder mit einem oder mehreren Fluoratomen substituiertes geradkettig oder verzweigtes (C₁ -C₆)-Alkyl, substituiert sein kann; geradkettig oder verzweigtes (C₁ -C6)-Alkoxy-(C₁ -C₆)-Alkyl, substituiertes oder unsubstituiertes Aryl-(C₁ -C₆)-alkyloxycarbonyl, geradkettig oder verzweigtes (C₁ -C₆)-Alkyloxycarbonyl, geradkettig oder verzweigtes (C₁ -C₆)-Alkylcarbonyl, (C₂ -C₆)-Alkenyl, (C₂ -C₆)-Alkinyl, oder geradkettiges oder verzweigtes Cyano-(C₁ -C₆)-Alkyl, bedeutet;
R₁ einen 2-, 3-, 4-, 5-, 6-, 7- oder 8-Chinolyl-Rest oder 2-, 3-, 4-, 5-, 6-, 7- oder 8-Chinolyl-(C₁ -C₄)-alkyl-Rest, wobei der (C₁ -C₄)-alkyl-Rest unsubstituiert oder ein- oder mehrfach gleich oder verschieden mit (C₁ -C₆)-Alkyl, Halogen oder Oxo (=O) substituiert sein kann und der 2-, 3-, 4-, 5-, 6-, 7- oder 8-Chinolyl-Rest unsubstituiert oder ein- bis sechsfach gleich oder verschieden mit Wasserstoff, (C₁-C₆)-Alkyl, Halogen, Nitro, Amino, Mono-(C₁-C₆)-Alkylamino, N,N-di-(C₁-C₆)-Alkylamino, Hydroxyl, (C₁-C₆)-Alkoxy, (C6-C14)-Aryl-(C1-C6)-alkyoxy, Carboxy, (C₁-C₆)-Alkoxycarbonyl, (C₁-C₆)-Alkoxycarbonylamino oder ein- oder mehrfach mit Halogen substituiertem (C₁ -C₆)-Alkyl, (C₆-C₁₄)-Aryl, oder (C₆-C₁₄)-Aryl-(C₁-C₆)-alkyl substituiert sein kann, ausgenommen mit (C1-C6)-Alkyl, Halogen, Nitro, Amino, und (C1-C6)-Alkylamino substituiertes oder unsubstituiertes 2-, 3-. 4- und 8-Chinolyl und 2-, 3- und 4-Chinolylmethyl, bei welchem die Ringkohlenstoffatome des Pyridylmethyl-Teils unsubstituiert oder mit (C1-C6)-Alkyl, (C1-C6)-Alkoxy, Nitro, Amino, und (C1-C6)-Alkoxycarbonylamino substituiert sind;
R₂ Wasserstoff, (C₁-C₆)-Alkyl, ein- oder mehrfach mit Halogen substituiertes (C₁-C₆)-Alkyl, ein- oder mehrfach mit Phenyl substituiertes (C₁-C₆)-Alkyl, wobei der Phenylrest unsubstituiert oder ein- bis fünffach gleich oder verschieden mit Halogen, (C₁-C₆)-Alkyl, (C₃-C₇)-Cycloalkyl, Carboxyl, (C₁-C₆)-Alkoxycarbonyl, ein- oder mehrfach mit Halogen substituiertem (C₁-C₆)-Alkyl, Hydroxy, (C₁-C₆)-Alkoxy, Phenyl-(C₁-C₆)-Alkoxy, Nitro, Amino, Mono-(C₁-C₆)-alkylamino, Di-(C₁-C₆)-alkylamino, Mono-(C₃-₆)-cycloalkylamino, Di-(C₃-₆)-cycloalkylamino, (C₁-C₆)-Acylamino, Phenyl-(C₁-C₆)-alkylamino, Arylcarbonylamino, Heteroarylcarbonylamino, (C₁-C₆)-Alkylsulfonamido, Arylsulfonamido, Maleinimido, Succinimido, Phthalimido, Benzyloxycarbonylamino (Z-Amino), tert.-Butoxycarbonylamino (BOC-Amino), 9-Fluorenylmethoxy-carbonylamino (Fmoc-Amino), Triphenylmethylamino (Tr-Amino), 2-(4'-Pyridyl)-ethoxycarbonylamino (Pyoc-Amino), Diphenylmethylsilyl-amino (DPMS-Amino), mit -NH-CH₂-COOH; -NH-CH(CH₃)-COOH; (CH₃)₂CH-CH₂-CH₂-CH(NH-)-COOH; H₃C-CH₂-CH(CH₃)-CH(NH-)-COOH;
HOH₂C-CH(NH-)-COOH; Phenyl-CH₂-CH(NH-)-COOH; (4-Imidazolyl)-CH₂-CH(NH-)-COOH; HN=C(NH₂)-NH-(CH₂)₃-CH(NH-)-COOH; H₂N-(CH₂)₄-CH(NH-)-COOH;
H₂N-CO-CH₂-CH(NH-)-COOH; HOOC-(CH₂)₂-CH(NH-)-COOH substituiert sein kann;
einen 2-, 3-, 4-, 5-, 6-, 7- und 8-Chinolyl-(C₁-C₆)-Alkyl-Rest, wobei der 2-, 3-, 4-, 5,-, 6- , 7- und 8-Chinolyl-Rest unsubstituiert oder ein- bis sechsfach gleich oder verschieden mit Halogen, (C₁-C₄)-Alkyl oder (C₁-C₄)-Alkoxy substituiert sein kann;
einen 2-, 3- und 4-Pyridyl-(C₁-C₆)-Alkyl-Rest, wobei der 2-, 3- und 4-Pyridyl-Rest unsubstituiert oder ein- bis vierfach gleich oder verschieden mit Halogen, (C₁-C₄)-Alkyl oder (C₁-C₄)-Alkoxy substituiert sein kann;
einen Arylcarbonyl-Rest, wobei der Aryl-Rest unsubstituiert oder ein- bis fünffach gleich oder verschieden mit Halogen, (C₁-C₆)-Alkyl, (C₃-C₇)-Cycloalkyl, Carboxyl, Cyano, (C₁-C₆)-Alkoxycarbonyl, ein- oder mehrfach mit Fluoratomen substituiertes (C₁-C₆)-Alkyl Hydroxyl, (C₁-C₄)-Alkoxy Aryl-(C₁-C₄)-Alkoxy, substituiert sein kann; bedeutet;
R₃ und R₄ können an die Indolkohlenstoffatome C-2, C-4, C-5, C-6 oder C-7 gebunden sein und unabhängig voneinder Wasserstoff, Hydroxy, (C₁-C₆)-Alkyl, (C₃-C₇)-Cycloalkyl, (C₁-C₆)-Alkylcarbonyl, (C₁-C₆)-Alkoxy, Halogen, Aryl-(C₁-C₄)-Alkoxy, Nitro, Amino, Mono-(C₁-C₄)-Alkyl-amino, Di-(C₁-C₄)-Alkylamino, (C₁-C₆)-Alkoxycarbonylamino, (C₁-C₆)-Alkoxycarbonylamino-(C₁-C₆)-alkyl, Cyano, geradkettiges oder verzweigtes Cyano-(C₁-C₆)-alkyl, Carboxyl, (C₁-C₄)-Alkoxycarbonyl, mit ein oder mehreren Fluoratomen substituiertes (C₁-C₄)-Alkyl, Carboxy-(C₁-C₆)-alkyl oder (C₁-C₆)-Alkoxycarbonyl-(C₁-C₆)-alkyl bedeuten;
Z₁ Sauerstoff oder Schwefel oder geminal verknüpftes Wasserstoff und Hydroxy bedeutet;
Z₂ Sauerstoff oder Schwefel bedeutet,
deren Tautomere, Stereoisomere, einschließlich der Diastereomere und Enantiomere, sowie die physiologisch verträglichen Salze davon.

2. Indolderivat nach Anspruch 1, **dadurch gekennzeichnet, dass** R, R₂, R₃, R₄, Z₁ und Z₂ die vorstehend beschriebenen Bedeutungen besitzen und R₁ für Chinolyl steht, welches ein oder mehrfach mit geradkettig oder verzweigtem (C₁-C₆-Alkyl) oder (C₁-C₆)-Alkoxy substituiert ist

3. Indolderivat nach einem der Ansprüche Anspruch 1 oder 2, **dadurch gekennzeichnet, dass** R, R₂, R₃, R₄, Z₁ und Z₂ die vorstehend beschriebenen Bedeutungen besitzen und R₁ für 2-Methyl-6-chinolyl steht.

4. Indolderivat nach einem der Ansprüche 1 bis 3, **dadurch gekennzeichnet, dass** R, R₂, R₃, R₄ Z₁ und Z₂ die vorstehend beschriebenen Bedeutungen besitzen und R₁ (2-Methyl)-6-chinolyl, R₂ 4-Chlorbenzyl, R₃ und R₄ jeweils Wasserstoff und Z₁ und Z₂ jeweils Sauerstoff bedeuten.

5. Arzneimittel, **dadurch gekennzeichnet, dass** es als wirksamen Bestandteil mindestens ein Indolderivat nach einem der Ansprüche 1 bis 4 gegebenenfalls zusammen mit üblichen pharmazeutisch verträglichen Hitfs-, Zusatz- und Trägersstoffen enthält.

6. Verwendung der Indolderivate gemäß der allgemeinen Formel (1) nach einem der Ansprüche 1 bis 5 zur Herstellung eines Arzneimittels zur Behandlung von Tumoren in Säugetieren.

7. Verfahren zur Herstellung von Indolderivaten gemäß der allgemeinen Formel (1) nach einem der Ansprüche 1 bis 4, **dadurch gekennzeichnet, dass** eine Indolvorstufe der allgemeinen Formel (2), 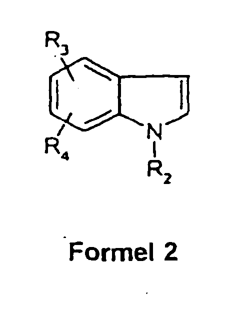 worin R₂, R₃ und R₄ die vorstehend genannten Bedeutungen besitzen, mit einer Verbindung der allgemeinen Formel (3) 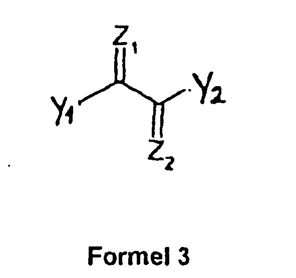 worin Z₁ und Z₂ die vorstehend genannte Bedeutung besitzen und Y₁ und Y₂ unabhängig voneinander für eine geeignete Abgangsgruppe wie Halogen, (C1-C6)-Alkoxy, -O-Tosyl, -O-Mesyl oder- N1-imidazol steht, und anschließend mit einem Amin der allgemeinen Formel (4) oder (5), 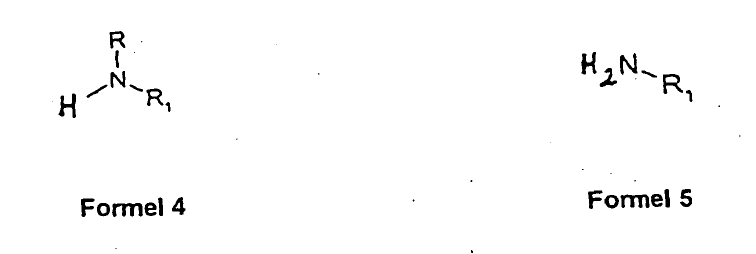 worin R und R₁ die vorstehend genannten Bedeutungen haben, unter Erhalt der gewünschten Verbindung der allgemeinen Formel (1) (mit Amin 4) oder der Verbindung der allgemeinen Formel (6) (mit Amin 5) 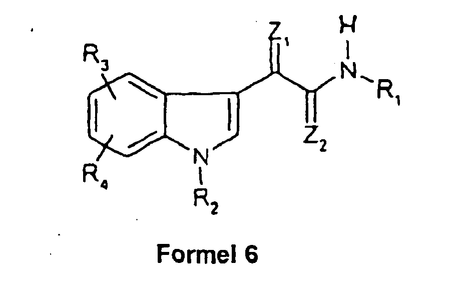 worin R₁, R₂, R₃, R₄, Z₁ und Z₂ die vorstehend genannten Bedeutungen besitzen, gegebenenfalls unter Verwendung von Verdünnungs- und Hilfsmitteln umgesetzt wird, wobei anschließend die Verbindung der allgemeinen Formel (6) mit einer Verbindung der allgemeinen Formel (7)
R-Y₃ (7)
worin R die vorstehend genannte Bedeutung besitzt und Y₃ für eine geeignete elektrophile Abgangsgruppe wie Halogen, (C1-C6)-Alkoxy, -O-Tosyl, -O-Mesyl oder -N1-imidazol steht, unter Erhalt der gewünschten Verbindung (1), wobei R nicht Wasserstoff bedeutet, gegebenenfalls unter Verwendung von Verdünnungs- und Hilfsmitteln umgesetzt werden kann.

## Claims

1. Indole derivatives of the general Formula 1 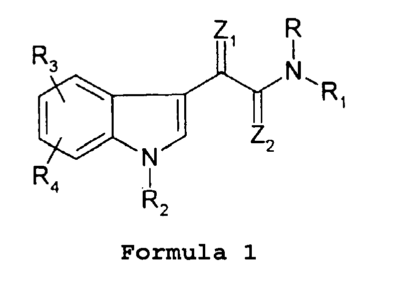 in which
R denotes hydrogen; straight-chain or branched (C₁-C₆) -alkyl; (C₁-C₆) -alkyl which is mono- or polysubstituted by aryl, where the aryl radical unsubstituted or mono- to penta- by identical or different substituents from the group consisting of halogen, (C₁-C₆) -alkyl, (C₃-C₇) -cycloalkyl, carboxyl, (C₁-C₆)-alkoxy-carbonyl, straight-chain or branched (C₁-C₆)-alkyl, which is substituted by one or more fluorine atoms, hydroxyl, straight-chain or branched (C₁-C₆)-alkoxy, benzyloxy or aryl-(C₁-C₆)-alkyl, where the aryl radical may be unsubstituted or mono- or pentasubstituted by identical or different substituents from the group consisting of (C₁-C₆)-alkyl, halogen and straight-chain or branched (C₁-C₆)-alkyl which is substituted by one or more fluorine atoms; straight-chain or branched (C₁-C₆) -alkoxy- (C₁-C₆)-alkyl, substituted or unsubstituted aryl-(C₁-C₆)-alkyloxycarbonyl, straight-chain or branched (C₁-C₆)-alkyloxycarbonyl, straight-chain or branched (C₁-C₆) -alkylcarbonyl, (C₂-C₆) -alkenyl, (C₂-C₆)-alkynyl, or straight-chain or branched cyano-(C₁-C₆) -alkyl;
R₁ denotes a 2-, 3-, 4-, 5-, 6-, 7- or 8-quinolyl radical or 2-, 3-, 4-, 5-, 6-, 7 or 8-quinolyl-(C₁-C₄)-alkyl radical, where the (C₁-C₄) -alkyl radical may be unsubstituted or mono- or polysubstituted by identical or different substituents from the group consisting of (C₁-C₆)-alkyl, halogen and oxo (=O) and the 2-, 3-, 4-, 5-, 6-, 7- or 8-quinolyl radical may be unsubstituted or mono- to hexasubstituted by identical or different substituents from the group consisting of hydrogen, (C₁-C₆)-alkyl, halogen, nitro, amino, mono-(C₁-C₆)-alkylamino, N,N-di- (C₁-C₆) -alkylamino, hydroxyl, (C₁-C₆)-alkoxy, (C₆-C₁₄) -aryl- (C₁-C₆)-alkyloxy carboxyl, (C₁-C₆) -alkoxycarbonyl, (C₁-C₆)-alkoxycarbonylamino or (C₁-C₆)-alkyl which is mono-or polysubstituted by halogen, (C₆-C₁₄)-aryl and (C₆-C₁₄) -aryl- (C₁-C₆) -alkyl, *except for* (C₁-C₆)-alkyl-, halogen-, nitro-, amino- and (C₁-C₆)-alkylamino-substituted or unsubstituted 2-, 3-, 4-and 8-quinolyl and 2-, 3- and 4-quinolylmethyl in which the ring carbon atoms of the pyridylmethyl moiety are unsubstituted or substituted by (C₁-C₆)-alkyl, (C₁-C₆)-alkoxy, nitro, amino and (C₁-C₆)-alkoxycarbonylamino;
R₂ denotes hydrogen, (C₁-C₆) -alkyl, (C₁-C₆) -alkyl which is mono- or polysubstituted by halogen, (C₁-C₆)-alkyl which is mono- or polysubstituted by phenyl, where the phenyl radical may be unsubstituted or mono- to pentasubstituted by identical or different substituents from the group consisting of halogen, (C₁-C₆)-alkyl, (C₃-C₇) -cycloalkyl, carboxyl, (C₁-C₆)-alkoxycarbonyl, (C₁-C₆)-alkyl which is mono- or polysubstituted by halogen, hydroxyl, (C₁-C₆) -alkoxy, phenyl- (C₁-C₆) -alkoxy, nitro, amino, mono- (C₁-C₆) -alkylamino, di- (C₁-C₆)-alkylamino, mono- (C₃-₆) -cycloalkylamino, di - (C₃ - ₆)-cycloalkylamino, (C₁-C₆) -acylamino, phenyl-(C₁-C₆)-alkylamino, arylcarbonylamino, heteroarylcarbonyl-amino, (C₁-C₆)-alkylsulfonamido, arylsulfonamido, maleinimido, succinimido, phthalimido, benzyloxy-carbonylamino (Z-amino), tert-butoxycarbonylamino (BOC-amino), 9-fluorenylmethoxycarbonylamino (Fmoc-amino), triphenylmethylamino (Tr-amino), 2-(4'-pyridyl)-ethoxycarbonylamino (Pyoc-amino), diphenylmethylsilylamino (DPMS-amino), by -NH-CH₂-COOH; -NH-CH (CH₃) -COOH; (CH₃) ₂ CH - CH₂ - CH₂-CH(NH-)-COOH; H₃C-CH₂-CH(CH₃)-CH(NH-)-COOH;
HOH₂C-CH(NH-)-COOH; phenyl-CH₂-CH (NH-) -COOH; (4-imidazolyl) -CH₂-CH (NH-) -COOH; HN=C (NH₂) -NH- (CH₂)₃-CH (NH-) -COOH; H₂N-(CH₂)₄-CH(NH-)-COOH;
H₂N-CO-CH₂-CH(NH-) -COOH; HOOC-(CH₂)₂-CH(NH-)-COOH;
a 2-, 3-, 4-, 5-, 6-, 7- and 8-quinolyl-(C₁-C₆)-alkyl radical, where the 2-, 3-, 4-, 5-, 6-, 7-and 8-quinolyl radical may be unsubstituted or mono- to hexasubstituted by identical or different substituents from the group consisting of halogen, (C₁-C₄)-alkyl or (C₁-C₄)-alkoxy;
a 2-, 3- and 4-pyridyl-(C₁-C₆)-alkyl radical, where the 2-, 3- and 4-pyridyl radical may be unsubstituted or mono- to tetrasubstituted by identical or different substituents from the group consisting of halogen, (C₁-C₄)-alkyl and (C₁-C₄)-alkoxy;
an arylcarbonyl radical, where the aryl radical may be unsubstituted or mono- to pentasubstituted by identical or different substituents from the group consisting of halogen, (C₁-C₆) -alkyl, (C₃-C₇)-cycloalkyl, carboxyl, cyano, (C₁-C₆)-alkoxycarbonyl, (C₁-C₆)-alkyl which is mono- or polysubstituted by fluorine atoms, hydroxyl, (C₁-C₄) -alkoxy, aryl- (C₁-C₄) -alkoxy;
R₃ and R₄ may be attached to the indole carbon atoms C-2, C-4, C-5, C-6 or C-7 and independently of one another denote hydrogen, hydroxyl, (C₁-C₆)-alkyl, (C₃-C₇) -cycloalkyl, (C₁-C₆) -alkylcarbonyl, (C₁-C₆)-alkoxy, halogen, aryl- (C₁-C₄) -alkoxy, nitro, amino, mono- (C₁-C₄) -alkylamino, di- (C₁-C₄) -alkylamino, (C₁-C₆) -alkoxycarbonylamino, (C₁-C₆)-alkoxycarbonylamino-(C₁-C₆)-alkyl, cyano, straight-chain or branched cyano-(C₁-C₆)-alkyl, carboxyl, (C₁-C₄) -alkoxycarbonyl, (C₁-C₄) -alkyl which is substituted by one or more fluorine atoms, carboxy- (C₁-C₆) -alkyl or (C₁-C₆)-alkoxycarbonyl-(C₁-C₆) -alkyl;
Z₁ denotes oxygen or sulfur or geminally attached hydrogen and hydroxyl;
Z₂ denotes oxygen or sulfur,
their tautomers, stereoisomers, including the diastereomers and enantiomers, and the physiologically acceptable salts thereof.

2. Indole derivative according to claim 1, **characterized in that** R, R₂, R₃, R₄, Z₁ and Z₂ have the meanings described above and R₁ represents quinolyl which is mono- or polysubstituted by straight-chain or branched (C₁-C₆-alkyl) or (C₁-C₆)-alkoxy.

3. Indole derivative according to either of claims 1 and 2, **characterized in that** R, R₂, R₃, R₄, Z₁ and Z₂ have the meanings described above and R₁ represents 2-methyl-6-quinolyl.

4. Indole derivative according to any of claims 1 to 3, **characterized in that** R, R₂, R₃, R₄, Z₁ and Z₂ have the meanings described above and R₁ denotes (2-methyl)-6-quinolyl, R₂ denotes 4-chlorobenzyl, R₃ and R₄ each denote hydrogen and Z₁ and Z₂ each denote oxygen.

5. Medicament, **characterized in that** it comprises, as active ingredient, at least one indole derivative according to any of claims 1 to 4, if appropriate together with customary pharmaceutically acceptable auxiliaries, additives and carriers.

6. Use of the indole derivatives of the general formula (1) according to any of claims 1 to 5 for preparing a medicament for treating tumors in mammals.

7. Process for preparing indole derivatives of the general formula (1) according to any of claims 1 to 4, **characterized in that** an indole precursor of the general formula (2) 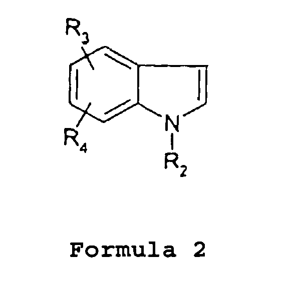 in which R₂, R₃ and R₄ have the meanings mentioned above is reacted with a compound of the general formula (3) 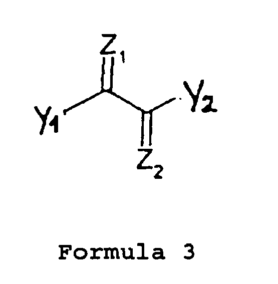 in which Z₁ and Z₂ have the meaning mentioned above and Y₁ and Y₂ independently of one another represent a suitable leaving group, such as halogen, (C1-C6)-alkoxy, -O-tosyl, -O-mesyl or -N1-imidazole, and then with an amine of the general formula (4) or (5) 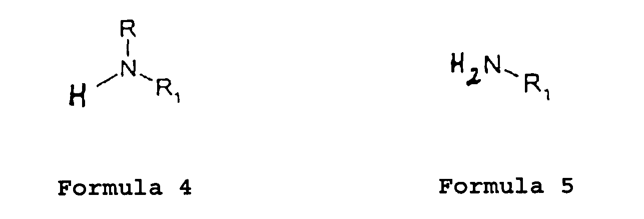 in which R and R₁ have the meanings mentioned above, giving the desired compound of the general formula (1) (with amine 4) or the compound of the general formula (6) (with amine 5) 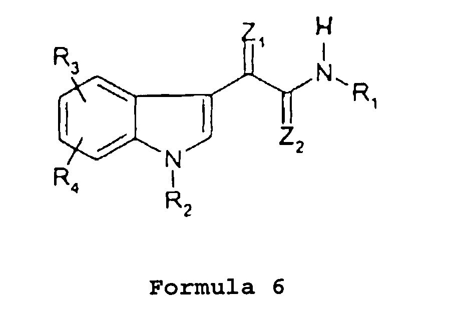 in which R₁, R₂, R₃, R₄, Z₁ and Z₂ have the meanings mentioned above, using, if appropriate, diluents and auxiliaries, where the compound of the general formula (6) may subsequently be reacted with a compound of the general formula (7)
R-Y3 (7)
in which R has the meaning mentioned above and Y₃ represents a suitable electrophilic leaving group, such as halogen, (C1-C6)-alkoxy, -O-tosyl, -O-mesyl or -N1-imidazole, giving the desired compound (1), where R does not denote hydrogen, using, if appropriate, diluents and auxiliaries.

## Revendications

1. Dérivés d'indole de formule générale 1 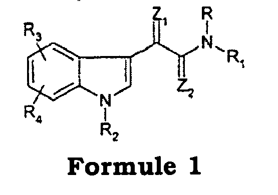 dans laquelle
R est un atome d'hydrogène, un groupe alkyle en C₁ à C₆ à chaîne droite ou ramifié ; un groupe alkyle en C₁ à C₆ substitué une ou plusieurs fois par un groupe aryle, le radical aryle pouvant être non substitué ou substitué une à cinq fois de manière identique ou différente par un groupe halogène, un groupe alkyle en C₁ à C₆, cycloalkyle en C₃ à C₇, carboxyle, alcoxycarbonyle en C₁ à C₆, un groupe alkyle en C₁ à C₆ à chaîne droite ou ramifié substitué par un ou plusieurs atomes de fluor, hydroxy, alcoxy en C₁ à C₆ à chaîne droite ou ramifié, benzyloxy ou aryle-alkyle en C₁ à C₆, le radical aryle pouvant être non substitué ou substitué une à cinq fois de manière identique ou différente par un groupe alkyle en C₁ à C₆, un groupe halogène ou un groupe alkyle en C₁ à C₆ à chaîne droite ou ramifié substitué par un ou plusieurs atomes de fluor ; un groupe alcoxy en C₁ à C₆-alkyle en C₁ à C₆ à chaîne droite ou ramifié, aryle-alcoxycarbonyle en C₁ à C₆ substitué ou non substitué, un groupe alkyloxycarbonyle en C₁ à C₆ à chaîne droite ou ramifié, un groupe alkylcarbonyle en C₁ à C₆ à chaîne droite ou ramifié, un groupe alcényle en C₂ à C₆, alcynyle en C₂ à C₆, ou bien un groupe cyano-alkyle en C₁ à C₆ à chaîne droite ou ramifié ;
R₁ représente un radical 2, 3, 4, 5, 6, 7 ou 8-quinolyle ou un radical 2, 3, 4, 5, 6, 7, ou 8-quinolyle-alkyle en C₁ à C₄, le radical alkyle en C₁ à C₄ pouvant être non substitué ou substitué une ou plusieurs fois de manière identique ou différente par un groupe alkyle en C₁ à C₆, halogène ou oxo (=O) et le radical 2, 3, 4, 5, 6, 7 ou 8-quinolyle pouvant être non substitué ou substitué une à six fois de manière identique ou différente avec de l'hydrogène, un groupe alkyle en C₁ à C₆, halogène, nitro, amino, mono-alkylamino en C₁ à C₆, N, N-di-alkylamino en C₁ à C₆, hydroxyle, alcoxy en C₁ à C₆, aryle en C₆ à C₁₄-alkyloxy en C₁ à C₆, carboxy, alcoxycarbonyle en C₁ à C₆, alcoxycarbonylamino en C₁ à C₆ ou alkyle en C₁ à C₆ substitué une ou plusieurs fois avec un halogène, aryle en C₆ à C₁₄ ou aryle en C₆ à C₁₄-alkyle en C₁ à C₆, à l'exception d'un groupe 2, 3, 4 et 8-quinolyle et 2, 3 et 4-quinolylméthyle substitué par un groupe alkyle en C₁ à C₆, halogène, nitro, amino et alkylamino en C₁ à C₆ ou non substitué, dans lequel les atomes de carbone cycliques de la partie pyridylméthyle ne sont pas substitués ou bien substitués avec un groupe alkyle en C₁ à C₆, alcoxy en C₁ à C₆, nitro, amino et alcoxycarbonylamino en C₁ à C₆ ;
R₂ représente un atome d'hydrogène, un groupe alkyle en C₁ à C₆, alkyle en C₁ à C₆ substitué une ou plusieurs fois par un groupe halogène, alkyle en C₁ à C₆ substitué une ou plusieurs fois par un groupe phényle, le radical phényle pouvant être non substitué ou substitué une à cinq fois de manière identique ou différente par un groupe halogène, un groupe alkyle en C₁ à C₆, cycloalkyle en C₃ à C₇, carboxyle, alcoxycarbonyle en C₁ à C₆, alkyle en C₁ à C₆ substitué une ou plusieurs fois par un groupe halogène, alcoxy en C₁ à C₆, de préférence méthoxy ou éthoxy, phényle-alcoxy en C₁ à C₆, nitro, amino, mono-alkylamino en C₁ en C₆, di-alkylamino en C₁ à C₆, mono-cycloalkylamino en C₃ à C₆, di-cycloalkylamino en C₃ à C₆, acylamino en C₁ à C₆, phényle-alkylamino en C₁ à C₆, arylcarbonylamino, hétéroarylcarbonylamino, alkylsulfonamido en C₁ à C₆, arylsulfonamido, maléinimido, succinimido, phtalimido, benzyloxycarbonylamino (Z-amino), tert-butoxycarbonylamino (BOC-amino), 9-fluorénylméthoxy-carbonylamino (Fmoc-amino), triphénylméthylamino (Tr-amino), 2-(4'-pyridyl)éthoxycarbonylamino (Pyoc-amino), diphénylméthylsilyl-amino (DPMS-amino), par un groupe -NH-CH₂-COOH ; -NH-CH(CH₃)-COOH ; (CH₃)₂CH-CH₂-CH₂-CH(NH-)-COOH ; H₃C-CH₂-CH(CH₃)-CH(NH-)-COOH ;
HOH₂C-CH(NH-)-COOH ; phényl-CH₂-CH(NH-)-COOH ; (4-imidazolyl)-CH₂-CH(NH-)-COOH ; HN=C(NH2)-NH-(CH₂)₃-CH(NH-)COOH ; H₂N-(CH₂)₄-CH(NH-)-COOH ;
H₂N-CO-CH₂-CH(NH-)-COOH ; HOOC-(CH₂)₂-CH(NH-)-COOH ;
un radical 2, 3, 4, 5, 6, 7 et 8-quinolyle-alkyle en C1 à C6, le radical 2, 3, 4, 5, 6, 7 et 8-quinolyle pouvant être non substitué ou substitué une à six fois de manière identique ou différente avec un groupe halogène, alkyle en C1 à C4 ou alcoxy en C1 à C4 ;
un radical 2, 3 et 4-pyridyle-alkyle en C1 à C6, le radical 2, 3 et 4-pyridyle pouvant être non substitué ou substitué une à quatre fois de manière identique ou différente avec un groupe halogène, alkyle en C 1 à C4 ou alcoxy en C 1 à C4 ;
un radical arylcarbonyle, le radical aryle pouvant être non substitué ou substitué une à cinq fois de manière identique ou différente par un groupe halogène, alkyle en C1 à C6, cycloalkyle en C3 à C7, carboxyle, cyano, alcoxycarbonyle en C1 à C6, alkyle en C1 à C6 substitué une ou plusieurs fois par des atomes de fluor, hydroxyle, alcoxy en C1 à C4, aryle-alcoxy en C1 à C4 ;
R3 et R4 peuvent être liés aux atomes de carbone de l'indole en position C-2, C-4, C-5 C-6 ou C-7, et représentent, indépendamment l'un de l'autre, un atome d'hydrogène, un groupe hydroxy, alkyle en C1 à C6, cycloalkyle en C3 à C7, alkylcarbonyle en C1 à C6, alcoxy en C1 à C6, halogène, aryle-alcoxy en C1 à C4, nitro, amino, mono-alkylamino en C1 à C4, di-alkylammino en C1 à C4, alcoxycarbonylamino en C1 à C6, alcoxycarbonylamino en C1 à C6-alkyle en C1 à C6, cyano, cyano-alkyle en C1 à C6 à chaîne droite ou ramifié, carboxyle, alcoxycarbonyle en C1 à C4, alkyle en C1 à C4 substitué par un ou plusieurs atomes de fluor, carboxy-alkyle en C 1 à C6 ou alcoxycarbonyle en C 1 à C6-alkyle en C 1 à C6 ;
Z1 représente un atome d'oxygène ou de soufre ou un atome d'hydrogène ou un groupe hydroxy associé de manière germinale ;
Z2 représente un atome d'oxygène ou de soufre,
leurs tautomères, stéréo-isomères, y compris les diastéréomères et les énantiomères, ainsi que les sels physiologiquement compatibles.

2. Dérivé d'indole selon la revendication 1,
**caractérisé en ce que**
R, R₂, R₃, R₄, Z₁ et Z₂ ont les significations décrites ci-dessus et R₁ représente un groupe quinolyle substitué une ou plusieurs fois par un groupe alkyle C₁ à C₆ ou alcoxy en C1 à C₆ à chaîne droite ou ramifié.

3. Dérivé d'indole selon l'une quelconque des revendications 1 ou 2,
**caractérisé en ce que**
R, R₂, R₃, R₄, Z₁ et Z₂ ont les significations décrites ci-dessus et R₁ représente un groupe 2-méthyl-6-quinolyle.

4. Dérivé d'indole selon l'une quelconque des revendications 1 à 3,
**caractérisé en ce que**
R, R₂, R₃, R₄, Z₁ et Z₂ ont les significations décrites ci-dessus, et R₁ représente un groupe (2-méthyl)-6-quinolyle, R₂ un groupe 4-chlorobenzyle, R₃ et R₄ respectivement un atome de carbone et Z₁ et Z₂ respectivement un atome d'hydrogène.

5. Médicament,
**caractérisé en ce qu'**
il contient comme constituant actif au moins un dérivé d'indole selon l'une quelconque des revendications 1 à 4, le cas échéant également des excipients, des additifs et des supports usuels pharmacologiquement compatibles.

6. Utilisation des dérivés d'indole de formule générale (1) selon l'une quelconque des revendications 1 à 5 pour fabriquer un médicament pour traiter les tumeurs chez les mammifères.

7. Procédé de production de dérivés d'indole de formule générale (1) selon l'une quelconque des revendications 1 à 4,
**caractérisé en ce qu'**
on convertit un précurseur de l'indole de formule générale (2) 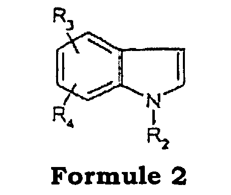 dans laquelle R₂, R₃ et R₄ ont les significations indiquées précédemment, avec un composé de formule générale (3) 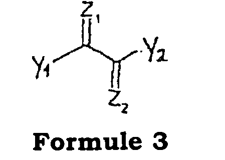 dans laquelle Z₁ et Z₂ ont la signification indiquée précédemment et Y₁ et Y₂, indépendamment l'un de l'autre, représentent un groupe partant approprié tel qu'un groupe halogène, alcoxy en C₁ à C6, -O-tosyle, O-mésyle ou -N1-imidazole, puis avec une amine de formule générale (4) ou (5), 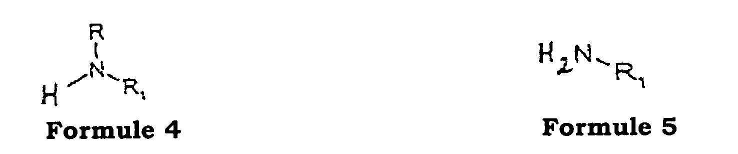 dans laquelle R et R₁ ont les significations indiquées précédemment, tout en obtenant le dérivé d'indole souhaité de formule générale (1) (avec l'amine 4) ou le composé de formule générale (6) (avec l'amine 5) 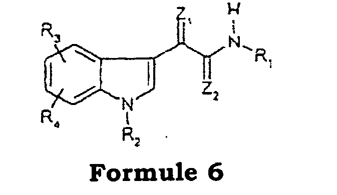 dans laquelle R₁, R₂, R₃, R₄, Z₁ et Z₂ ont les significations indiquées précédemment, le cas échéant en utilisant des agents diluants et des excipients, le composé de formule générale (6) pouvant ensuite être converti avec un composé de formule générale (7)
R-Y₃ (7)
dans laquelle R a la signification indiquée précédemment et Y3 représente un groupe partant électrophile approprié tel qu'un groupe halogène, alcoxy en C₁ à C₆, -O-tosyle, -O-mésyle ou -N1-imidazole, tout en obtenant le composé (1) souhaité, R ne représentant pas un atome d'hydrogène, le cas échéant en utilisant des agents diluants et des excipients.
